(19) Europäisches Patentamt
European Patent Office
Office européen des brevets

(11) Numéro de publication : **0 408 448 B1**

(12) **FASCICULE DE BREVET EUROPEEN**

(45) Date de publication du fascicule du brevet : **05.05.93 Bulletin 93/18**

(51) Int. Cl.$^5$ : **C07C 271/22,** A61K 7/48

(21) Numéro de dépôt : **90401995.7**

(22) Date de dépôt : **11.07.90**

(54) **Nouveaux dérivés d'uréthanne, leur préparation et leur application notamment comme agents hydratants dans des compositions cosmétiques ou pharmaceutiques destinées au traitement des peaux sèches.**

(30) Priorité : **11.07.89 FR 8909328**

(43) Date de publication de la demande : **16.01.91 Bulletin 91/03**

(45) Mention de la délivrance du brevet : **05.05.93 Bulletin 93/18**

(84) Etats contractants désignés : **AT BE CH DE DK ES FR GB GR IT LI NL SE**

(56) Documents cités :
**BE-A- 638 521**
**FR-A- 2 192 795**
**US-A- 4 732 892**

(73) Titulaire : **L'OREAL**
**14, Rue Royale**
**F-75008 Paris (FR)**

(72) Inventeur : **Philippe, Michel**
**3, rue de l'Aubépine**
**F-92160 Antony (FR)**
Inventeur : **Sebag, Henri**
**26 rue Erlanger**
**F-75016 Paris (FR)**
Inventeur : **Vanlerberche, Guy**
**Rue du Général de Gaulle**
**F-77410 Montjay la tour (FR)**
Inventeur : **De Rigal, Jean**
**Chemin de la Rosée - Gressy**
**F-77410 Claye Souilly (FR)**

(74) Mandataire : **Tonnellier, Jean-Claude et al**
**Cabinet Nony & Cie. 29, rue Cambacérès**
**F-75008 Paris (FR)**

EP 0 408 448 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux uréthannes dérivés d'acides aminés, leur préparation et leur application, notamment comme agents hydratants ou comme agents tensioactifs doux dans des compositions cosmétiques ou pharmaceutiques.

L'invention a plus précisément pour objet des dérivés d'uréthanne de formule générale :

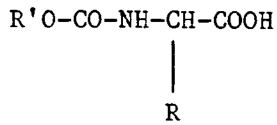

(I)

dans laquelle R représente un groupement

$-CH_2OH$, $-CHOH-CH_3$ ou $-(CH_2)_3-NH-CO-NHY$,

Y représentant -H ou -CO-OR',

R' représente un groupement alkyle linéaire ou ramifié, éventuellement insaturé, ayant de 8 à 24 atomes de carbone, ou un groupement cycloalkyle monocyclique substitué par un alkyle, dont le nombre total des atomes de carbone est égal ou supérieur à 10,

ainsi que les sels des composés de formule I, et les mélanges des composés de formule I et/ou de leurs sels.

Parmi les sels des composés de formule I, on citera les sels compatibles avec l'application sur la peau, et notamment les sels métalliques tels que les sels de sodium, de zinc, de magnésium, les sels d'aluminium et les sels cuivriques ou les sels de cations organiques tels que les sels d'un ammonium quaternaire de formule :

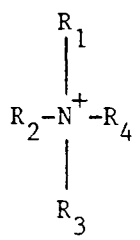

dans laquelle $R_1$, $R_2$, $R_3$ et $R_4$ représentent indépendamment un groupement $-CH_3$, $-CH_2-C_6H_5$ ou $-CH_2-CH_2OH$.

Dans les composés de formule I, R' représente notamment un groupement alkyle ayant de 8 à 24 atomes de carbone, ou un groupement alkyle mono- ou poly-insaturé tel que par exemple un radical alkyle mono-insaturé en $C_{18}$, un groupement cycloalkyle ayant par exemple 5 ou 6 chaînons substitué par un alkyle dont le nombre total d'atomes est égal ou supérieur à 10, comme le groupement (t-butyl) cyclohexyle.

L'invention a également pour objet un procédé de préparation des composés de formule I.

Ce procédé est caractérisé par le fait que l'on fait réagir un sel d'un acide aminé (D-,L- ou DL) choisi parmi la sérine, la thréonine, et la citrulline avec un composé de formule II

X-CO-OR' (II)

X représentant un halogène ou un groupement 1-imidazolyle, dans un solvant approprié, que l'on isole selon les méthodes connues le dérivé d'uréthanne correspondant de formule I formé et que, si désiré, on transforme selon les méthodes connues ledit dérivé d'uréthanne en sel correspondant.

Le sel de départ est par exemple un sel de métal alcalin ou un sel d'amine telle que la triéthylamine.

La réaction entre le sel d'acide aminé et l'halogénoformiate (de préférence le chloroformiate) peut être effectuée à température ambiante dans un solvant classique tel qu'un mélange eau-tétrahydrofuranne, eau-dioxanne ou eau-pyridine.

La réaction entre le sel d'acide aminé et le dérivé d'imidazole de formule II peut être effectué par exemple dans le N,N-diméthylformamide ou le N,N-diméthylacétamide à une température de 20 à 100°C, par exemple à 60°C, en présence d'un catalyseur basique tel que le t-butanolate de potassium ou l'imidazolidure de sodium.

On voit, d'après la formule I, que le procédé qui vient d'être mentionné permet la création d'une fonction uréthanne dont l'azote provient de la fonction amine de l'acide aminé. Dans le cas de la citrulline, une fonction uréine peut également être créée (voir la définition de Y) par réaction du composé de formule II avec la fonction

uréido de la citrulline. Cette dernière réaction n'a évidemment lieu qu'en présence d'une quantité suffisante de composé de formule II.

Les composés de formule I et leurs sels peuvent être utilisés chez l'homme comme agents d'hydratation de la peau, et permettent de conserver ou de restaurer la souplesse de la peau, son élasticité, sa résistance aux mouvements du corps et sa fonction de barrière à l'entrée des substances toxiques. On sait que les compositions cosmétiques ou dermopharmaceutiques destinées à hydrater la peau (préparations hydratantes) sont utilisées chez les personnes ayant une peau dite sèche. Ce phénomène est caractérisé généralement par une peau ayant un taux d'évaporation nettement plus élevé que celui d'une peau saine, par une perte de l'élasticité cutanée et par la formation de rides. Il peut être provoqué notamment par des troubles pathologiques de la kératinisation, par le vieillissement ou par l'exposition excessive au soleil ou à divers agents extérieurs (détergents, savons, solvants, atmosphère sèche, etc...). Le phénomène peut affecter toutes les parties du corps, et particulièrement le visage, le cou et les mains.

Les dérivés d'uréthanne de l'invention ont également des propriétés tensio-actives et peuvent être utilisés comme détergents doux dans des compositions cosmétiques ou pharmaceutiques pour la peau ou les cheveux.

En outre, certains dérivés d'uréthanne de l'invention peuvent former dans l'eau ou dans les solvants aqueux des structures vésiculaires capables de piéger et de retenir des substances hydrophobes ou hydrophiles, et peuvent être utilisés comme véhicules d'ingrédients actifs lipophiles ou hydrophiles, notamment dans des compositions cosmétiques ou pharmaceutiques.

La présente invention a donc également pour objet une composition cosmétique ou pharmaceutique caractérisée par le fait qu'elle comprend comme ingrédient actif au moins un dérivé d'uréthanne tel que défini précédemment, dans un véhicule compatible avec l'application sur la peau et/ou sur les cheveux.

Dans les compositions de l'invention, les dérivés d'uréthanne sont présents à une concentration pouvant aller de 0,1 à 15%, et de préférence de 0,5 à 5% en poids par rapport au poids total de la composition.

Les compositions de l'invention sont notamment des solutions ou émulsions du type lotions, moussantes ou non; des émulsions de consistance liquide ou semi-liquide du type laits, obtenues par dispersion d'une phase grasse dans une phase aqueuse ou inversement; des suspensions ou émulsions de consistance molle du type crèmes; ou des préparations solides telles que des sticks ou des pains de nettoyage.

Les véhicules présents dans les compositions de l'invention sont les véhicules classiques utilisés dans ce genre de composition. Il s'agit par exemple de l'eau et des solvants organiques compatibles avec l'application cutanée tels que par exemple l'acétone, l'alcool isopropylique, l'alcool éthylique, les triglycérides d'acides gras en $C_6$-$C_{24}$, les éthers de glycol tels que les éthers d'alkyle inférieur de mono-ou di-alkylène glycol, l'alkylène ayant par exemple 2 à 4 atomes de carbone. On peut également utiliser comme solvants les esters de polyalkylèneglycol et d'acides à chaîne courte en $C_1$-$C_4$, ou encore des silicones volatils.

Les compositions peuvent aussi contenir le cas échéant des corps gras, notamment des huiles, naturelles ou synthétiques.

Les compositions de l'invention peuvent également renfermer des agents épaississants ou gélifiants tels que la cellulose ou des dérivés de cellulose, par exemple à raison de 0,5 à 20% en poids par rapport au poids total de la composition. Les agents épaississants peuvent encore être constitués par des polymères acryliques, des alginates, des gommes, telles que la gomme de xanthane, de guar, de caroube, la gomme arabique ou bien des polyéthylèneglycols, des bentonites et des montmorillonites.

Les compositions de l'invention peuvent en outre contenir d'autres agents hydratants ou humectants connus, tels que la glycérine, la triacétine, ou plus généralement d'autres ingrédients actifs tels que des agents actifs contre le vieillissement de la peau.

Les compositions de l'invention peuvent également contenir des adjuvants usuels tels que des agents anti-oxydants, des agents conservateurs, des parfums, des colorants, etc...

Parmi les anti-oxydants, on citera les tert-butylhydroxyquinone, le butylhydroxyanisole, le butylhydroxytoluène et l'alpha-tocophérol et ses dérivés.

Les compositions de l'invention se présentent notamment sous la forme de crèmes, de laits, de gels, de lotions éventuellement épaissies, de solutions moussantes pour la douche ou le bain, de tampons imprégnés, de pommades, de sticks ou sous forme de pains ou de masques hydratants.

Les compositions pour cheveux sont notamment des shampooings dans lesquels le dérivé d'uréthanne peut être associé à d'autres agents tensio-actifs tels que les tensio-actifs anioniques, cationiques, non ioniques, amphotères ou leurs mélanges.

Les compositions de l'invention peuvent également se présenter sous la forme de solutions ou d'émulsions contenant des dérivés d'uréthanne tels que définis ci-dessus sous la forme vésiculaire, les vésicules pouvant alors servir d'agents d'encapsulation, d'ingrédients actifs lipophiles ou hydrophiles tels que l'acide rétinoïque des agents filtrant l'ultraviolet, et notamment d'autres agents hydratants.

Toutes ces compositions sont préparées selon les méthodes usuelles.

L'invention a également pour objet l'utilisation d'un dérivé d'uréthanne tel que défini précédemment comme ingrédient actif dans la préparation d'une composition cosmétique ou pharmaceutique destinée au traitement ou au soin des peaux sèches.

L'invention a en outre pour objet un procédé de traitement cosmétique destiné notamment à améliorer l'aspect et l'élasticité de la peau des personnes à peau sèche, ou destiné à prévenir l'apparition des troubles esthétiques provoques par ce phénomène de peau sèche, caractérisé par le fait que l'on applique sur la peau des parties du corps concernées, y compris éventuellement le cuir chevelu, une composition cosmétique telle que définie précédemment.

L'application des compositions de l'invention est effectuée selon les méthodes usuelles.

Le procédé de traitement cosmétique de l'invention est applicable notamment dans les cas de dermatose sèche, d'ichtiosis, de xéroses, etc....

Les exemples suivants illustrent l'invention sans toutefois la limiter :

EXEMPLE 1

PREPARATION DE LA N-HEXADECYLOXYCARBONYL (D,L)-SERINE

On dissout 15g de (D,L)-sérine dans 50ml d'eau à température ambiante puis on ajoute 143 méq d'hydroxyde de sodium sous la forme d'une solution aqueuse à 10 méq/g; on agite pendant 20 min puis on ajoute 50ml de tétrahydrofuranne sous forte agitation. On ajoute ensuite goutte à goutte (en 1 heure) 48,7g de chloroformiate de cétyle tout en maintenant le pH du milieu à une valeur supérieure à 9, ce qui nécessite l'ajout de 143 méq d'hydroxyde de sodium sous la forme d'une solution aqueuse à 10 méq/g. On laisse ensuite le milieu sous forte agitation pendant 1 heure tout en continuant de maintenir un pH supérieur à 9. On ajoute alors 250ml d'une solution aqueuse à 10% d'acide chlorhydrique puis on extrait l'uréthanne formé à l'aide d'acétate d'éthyle (250ml). La phase organique est séchée sur sulfate de sodium, les solvants sont évaporés sous vide et le résidu sec obtenu est cristallisé dans l'heptane. On obtient 32g (60% de rendement) de N-hexadécyloxycarbonyl (D,L)-sérine sous la forme d'une poudre blanche.

Le produit, recristallisé dans un mélange éther isopropylique/heptane, fond à 89°C.

Analyse élémentaire : $C_{20} H_{39} NO_5$ , 0,5 $H_2O$ ; M = 382,5

| | C | H | N |
|---|---|---|---|
| calculé % | 62,8 | 10,54 | 3,66 |
| trouvé % | 63,32 | 10,55 | 3,67 |

Le spectre de RMN du $^{13}C$ ($CDCl_3/CD_3OD$ - T.M.S.) est conforme à la structure indiquée avec un déplacement du carbonyle de l'uréthanne à 157,31 p.p.m. et un déplacement du carbonyle de l'acide à 172,98 p.p.m.

De façon analogue, on prépare le dérivé correspondant de la thréonine et de la citrulline.

EXEMPLE 2

PREPARATION DE LA N-(ETHYL-2-HEXYLOXYCARBONYL) (D,L)-SERINE

On dissout 42g de (D,L)-sérine dans 100ml d'eau puis on ajoute 400 méq de NaOH à l'aide d'une solution aqueuse à 10 méq/g (40g). On agite pendant 20 min. puis on ajoute 100ml de tétrahydrofuranne tout en augmentant l'agitation. On ajoute ensuite goutte à goutte, en 1 heure, 77,4g de chloroformiate d'éthyl-2-hexyle tout en maintenant le pH du milieu à une valeur supérieure à 9, ce qui nécessite de rajouter, au cours de cette addition 400 meq de soude sous forme d'une solution à 10 meq/g. On laisse le milieu sous forte agitation pendant 1 heure tout en continuant de maintenir un pH supérieur à 9. On ajoute alors 500ml d'une solution aqueuse à 10% d'acide chlorhydrique puis l'uréthanne formé est extrait à l'acétate d'éthyle (500ml). La phase organique est séchée sur sulfate de sodium, les solvants sont évaporés sous vide et le liquide visqueux obtenu est cristallisé dans l'heptane pour obtenir 60g (60% de rendement) de N-éthyl-2-hexyloxycarbonyl (D,L)-sérine sous la forme d'une poudre blanche.

Le produit obtenu, recristallisé dans un mélange éther-isopropylique/heptane fond à 37°C.

analyse élémentaire : $C_{12} H_{23} NO_5$, 0,5 $H_2O$ ; M = 270,3

| | C | H | N |
|---|---|---|---|
| calculé % | 53,32 | 8,95 | 5,18 |
| trouvé % | 52,98 | 8,93 | 5,04 |

Le spectre de R.M.N. du $^{13}C$ ($CDCl_3/CD_3OD$ - T.M.S.) est conforme à la structure indiquée avec un déplacement du carbonyle de l' uréthanne à 157,42 p.p.m. et un déplacement du carbonyle de l'acide à 172,96 p.p.m.

De façon analogue, on prépare le dérivé correspondant de la thréonine et de la citrulline.

## EXEMPLE 3

### PREPARATION DU SEL DE TRIETHANOLAMINE DE LA N-HEXADECYLOXY-CARBONYL (D,L)-SERINE

On chauffe à 65°C pendant 1 heure un mélange de 223,8g de N-hexadecyloxycarbonyl (D,L)-sérine et 89,4g de triéthanolamine dans 400ml d'isopropanol. Le solvant est ensuite évaporé sous vide pour obtenir 313g du sel attendu sous forme cristalline.

F = 73 - 75°C

Analyse élémentaire : $C_{26} H_{54} N_2 O_8$ ; M = 522,7

| | C | H | N |
|---|---|---|---|
| Calculé % | 59,74 | 10,41 | 5,36 |
| Trouvé % | 59,26 | 10,31 | 5,37 |

Le spectre de R.M.N. du $^{13}C$ ($CD_3OD$ - T.M.S.) est conforme à la structure attendue.

De façon analogue, on prépare le dérivé correspondant de la thréonine et de la citrulline.

## EXEMPLE 4

### PREPARATION DU SEL DE TRIETHANOLAMINE DE LA N-(ETHYL-2-HEXYLOXYCARBONYL) (D,L)-SERINE

Ce sel est obtenu de façon analogue à celle décrite à l'exemple 3. Il est obtenu sous forme d'une cire ambrée.

Analyse élémentaire : $C_{18} H_{38} N_2 O_8$, 0,5 $H_2O$ ; M = 419,7

| | C | H | N |
|---|---|---|---|
| Calculé % | 51,51 | 9,36 | 6,70 |
| Trouvé % | 51,69 | 9,36 | 6,73 |

Le spectre de R.M.N. du $^{13}C$ ($CD_3OD$ - T.M.S.) est conforme à la structure attendue.

De façon analogue, on prépare le dérivé correspondant de la thréonine et de la citrulline.

## EXEMPLE 5

### N-DODECYLOXYCARBONYL (D,L)-SERINE

- Le mode opératoire est analogue à celui décrit à l'exemple 1.

Le produit obtenu, recristallisé dans l'heptane, fond à 92°C.

Analyse élémentaire : $C_{16}H_{31}NO_5$, 0,5$H_2O$ ; M = 326

| | C | H | N |
|---|---|---|---|
| Calculé % | 58,87 | 9,88 | 4,29 |
| Trouvé % | 58,15 | 9,97 | 3,84 |

Le spectre de RMN du $^{13}C$ ($CDCL_3$ 4/ $CD_3OD$ 1-T.M.S) est conforme à la structure indiquée avec un déplacement du carbonyle de l'uréthanne à 157,36 et un déplacement du carbonyle de l'acide à 173,05 p.p.m.

EXEMPLE 6

N P-TERT-BUTYLCYCLOHEXYLOXYCARBONYL (D,L)-CITRULLINE

Le mode opératoire est analogue à celui de l'exemple 1
Le produit recristallisé dans un mélange eau/heptane fond à 79°C.
- Analyse élémentaire : $C_{17}H_{31}N_3O_5$,$0,5H_2O$ ; M = 366,5

| | C | H | N |
|---|---|---|---|
| Calculé % | 55,72 | 8,81 | 11,47 |
| Trouvé % | 56,06 | 8,71 | 11,53 |

Le spectre de R.M.N du $^{13}C$ ($CD_3OD$-T.M.S) est conforme à la structure indiquée avec un déplacement du carbonyle de l'uréthanne à 158,5 p.p.m., un déplacement du carbonyle de l'uréido à 162,2 p.p.m et un déplacement du carbonyle de l'acide à 175,9 p.p.m.

EXEMPLE 7

SEL DE SODIUM DE LA N-HEXADECYLOXYCARBONYL (D,L)-SERINE

Ce produit est obtenu par neutralisation du produit de l'exemple 1 par la soude dans un mélange isopropanol/eau à 60°C.
F = 155°C (isopropanol/eau)
- Analyse élémentaire : $C_{20}H_{38}NO_5Na$ ; M = 395,5

| | C | H | N | O |
|---|---|---|---|---|
| Calculé % | 60,73 | 9,63 | 3,54 | 20,23 |
| Trouvé % | 60,87 | 9,65 | 3,43 | 20,76 |

EXEMPLE 8

SEL DE SODIUM DE LA N-ETHYL-2-HEXYLOXYCARBONYL (D,L)-SERINE

Ce produit est obtenu par neutralisation du produit de l'exemple 2 par la soude dans un mélange isopropanol/eau à 20°C.
F = 85°C (isopropanol/eau)
Analyse élémentaire : $C_{12}H_{22}NO_5Na$ ; M = 283,3

| | C | H | N | O |
|---|---|---|---|---|
| Calculé % | 50,87 | 7,83 | 4,94 | 28,24 |
| Trouvé % | 50,91 | 7,68 | 4,76 | 28,41 |

ETUDE COMPARATIVE DE L'INFLUENCE DES DERIVES D'URETHANNE DE L'INVENTION SUR L'ELASTICITE D'UN STRATUM CORNEUM DELIPIDE

Cette étude est effectuée à l'aide de l'appareillage permettant de déterminer l'élasticité de la peau, décrit par L.RASSENEUR, J.DE RIGAL, et J.L. LEVEQUE, Int. J. of Cosm. Sci. 4, 247-260 (1982).

On a mesuré l'élasticité d'un stratum corneum préalablement délipidé sur lequel on a appliqué des solutions à 3% du produit étudié dans un mélange 2:1 chloroforme /méthanol. En délipidant préalablement le stratum corneum, celui-ci se rigidifie et devient donc un excellent modèle pour l'étude de l'effet des produits étudiés dans le domaine de l'augmentation de l'élasticité du stratum corneum. Les mesures sont effectuées 20 heures après le traitement.

Les résultats sont exprimés en pourcentage moyen de variation du module d'élasticité entre les valeurs, après application et avant application, du module d'élasticité du stratum corneum délipidé.

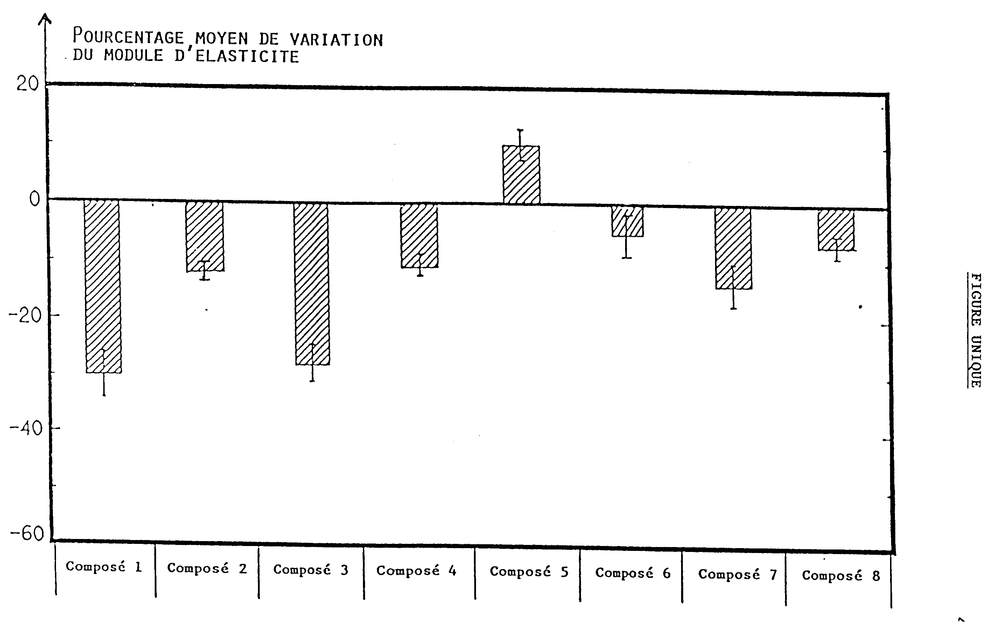

Les résultats obtenus avec le produit de l'exemple 2 (appelé composé 5) et divers produits de comparaison connus, de formule voisine, sont présentés sur la figure annexée.

Sur la figure :

- le composé 1 est la N-oléoyl (D,L)-sérine
- le composé 2 est la N-éthyl-2-hexanoyl (D,L)-sérine
- le composé 3 est la N-dodécanoyl (D,L)-sérine
- le composé 4 est la N-hexadécanoyl (D,L)-sérine
- le composé 5 est la N-éthyl-2-hexyloxycarbonyl (D,L)-sérine
- le composé 6 est la N-dodécyloxycarbonyl-glycine
- le composé 7 est la N-linoléoyl (D,L)-sérine
- le composé 8 est la N-octanoyl (D,L)-sérine

EXEMPLES DE COMPOSITIONS COSMETIQUES

Dans ces exemples, les produits désignés par des marques commerciales sont les suivants :

KLUCEL H : Hydroxypropylcellulose, vendue par la société HERCULES.

TWEEN 60 : Monostéarate de sorbitan polyoxyéthyléné à 20 moles d'oxyde d'éthylène, vendu par la société ICI AMERICAS.

CARBOPOL 940 : Polymère acrylique réticulé avec un agent polyfonctionnel, vendu par la société GOODRICH.

EXEMPLE A : GELS

On a préparé, par mélange des ingrédients, trois compositions sous forme de gels ayant les formulations suivantes :

a) N-éthyl-2-hexyloxycarbonyl (D,L)-sérine ... 3g
KLUCEL H .................................. 1g
Isopropanol .............................. 48g
Eau ...................................... 48g

b) N-hexadécyloxycarbonyl (D,L)-sérine ...... 3g
KLUCEL H .................................. 1g
Isopropanol .............................. 75g
Eau ...................................... 21g

c) Sel de triéthanolamine du N-éthyl-2 hexyloxycarbonyl (D,L)-sérine .................... 2g
KLUCEL H ................................ 1g
Ethanol ................................. 49g
Eau ..................................... 48g

On obtient des compositions analogues en remplaçant le dérivé de sérine par le dérivé correspondant de la thréonine ou de la citrulline.

EXEMPLE B- LOTIONS

On a préparé deux lotions ayant les formulations suivantes :

a) N-éthyl-2-hexyloxycarbonyl (D,L)-sérine .... 3g
Isopropanol............................... 49g
Eau....................................... 48g

b) Sel de triéthanolamine du N-hexadécyloxycarbonyl-D-,L-sérine ...................... 2g
Isopropanol............................... 50g
Eau....................................... 48g

On obtient des compositions analogues en remplaçant le dérivé de sérine par le dérivé correspondant de la thréonine ou de la citrulline.

EXEMPLE C - CREME DE SOINS POUR LA PEAU

On a préparé une crème ayant la composition suivante :
- N-hexadécyloxycarbonyl (D,L)-sérine 3 g
- Stéarate de glycérol 2 g
- TWEEN 60 1 g
- Alcool cétylique 0,5 g
- Acide stéarique 1,4 g
- Triéthanolamine 0,7 g
- CARBOPOL 940 (neutralisé par la triéthanolamine) 0,4 g
- Fraction liquide de graisse de karité 12 g
- Perhydrosqualène de synthèse 12 g
- Anti-oxydant 0,05g
- Parfum 0,5 g
- Conservateur 0,3 g
- Eau qsp 100 g

L'anti-oxydant est un mélange de butylhydroxytoluène et de butylhydroxyanisole.

L'agent conservateur est un mélange de p-hydroxybenzoates de méthyle, d'éthyle, de propyle, de butyle et d'isobutyle.

Cette crème (émulsion huile dans l'eau) est préparée de la façon suivante :

On ajoute le CARBOPOL 940, neutralisé par la triéthanolamine à une partie de l'eau (85 à 90%) et on chauffe à 75-80°C. On ajoute alors en agitant la phase grasse (stéarate de glycérol, TWEEN 60, acide stéarique, alcool cétylique, fraction liquide de graisse de karité, perhydrosqualène, anti-oxydant) portée à la même température, dans laquelle on a ajouté en dernier lieu la triéthanolamine. Après 10 minutes d'agitation, on ajoute le dérivé de sérine et le conservateur, dissous dans le restant de l'eau. Au bout de 10 minutes supplémentaires, on ajoute le parfum puis on arrête l'agitation et on refroidit jusqu'à la température ambiante (25°C).

On obtient une composition analogue en remplaçant le dérivé de sérine par le dérivé correspondant de la thréonine ou de la citrulline.

EXEMPLE D : CREME DE SOINS POUR LA PEAU

On a préparé une crème ayant la composition suivante :

- N-éthyl-2 hexyloxycarbonyl (D,L)-sérine ...... 4 g
- Monoisostéarate de sorbitan ................ 5 g
- Cire microcristalline ...................... 1 g
- Huile de vaseline.......................... 15g
- Huile de germes de maïs..................... 4g
- Mélange* d'esters d'acides gras en $C_8$-$C_{10}$ et d'alcools gras $C_{12}$-$C_{18}$ ................ 1g
- Gel** de montmorillonite modifié et d'huile neutre (triglycérides d'acides caprylique et caprique)................................ 5 g
- Propylène glycol........................... 3 g
- Anti-oxydant ............................... 0,1g
- Conservateur ............................... 0,3g
- Eau...............................q.s.p.... 100g

* Commercialisé par HENKEL sous la dénomination commerciale "CETIOL-LC DEO"

** Commercialisé par DYNAMIT NOBEL sous la dénomination commerciale "MIGLYOL-GEL"

Cette crème (eau-dans-l'huile) est préparée de façon analogue à celle décrite à l'exemple C.

Les agents anti-oxydants et conservateurs sont les mêmes qu'à l'exemple C.

On obtient une composition analogue en remplaçant le dérivé de sérine par le dérivé correspondant de la thréonine ou de la citrulline.

EXEMPLE E : LAIT CORPOREL

On a préparé un lait (émulsion fluide) ayant la composition suivante :

- Sel de triéthanolamine de N-hexadécyloxycarbonyl (D, L)-sérine 5 g
- Stéarate de glycérol 2 g
- TWEEN 60 1 g
- Acide stéarique 1,4 g
- triethanolamine 0,7 g
- CARBOPOL 940 (neutralisé par la triéthanolamine) 0,2 g
- Huile d'amandes douces 3 g
- Huile de vaseline 8 g
- Anti-oxydant 0,05g
- Conservateur 0,3 g

- Eau q.s.p 100 g

Ce lait corporel est préparé de façon analogue à celle décrite à l'exemple C.

On obtient une composition analogue en remplaçant le dérivé de sérine par le dérivé correspondant de la thréonine ou de la citrulline.

## Revendications

1. Dérivés d'uréthanne de formule générale :

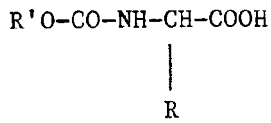

(I)

dans laquelle R représente un groupement

$-CH_2OH$, $-CHOH-CH_3$ ou $-(CH_2)_3-NH-CO-NHY$,

Y représentant -H ou COOR',

R' représente un groupement alkyle linéaire ou ramifié, éventuellement insaturé, ayant de 8 à 24 atomes de carbone, ou un groupement cycloalkyle monocyclique substitué par un alkyle dont le nombre total des atomes de carbone est égal ou supérieur à 10,

ainsi que les sels des composés de formule I, et les mélanges des composés de formule I et/ou de leurs sels.

2. Dérivés d'uréthanne selon la revendication 1, caractérisés par le fait que lesdits sels sont des sels métalliques ou des sels de cations organiques compatibles avec l'application sur la peau.

3. Dérivés d'uréthanne selon la revendication 2, caractérisés par le fait que lesdits sels sont choisis parmi les sels de sodium, de zinc, de magnésium et d'aluminium, et les sels cuivriques.

4. Dérivés d'uréthanne selon la revendication 2, caractérisés par le fait que lesdits sels sont des sels d'ammonium quaternaires.

5. Dérivés d'uréthanne selon la revendication 4, caractérisés par le fait que lesdits sels d'ammonium quaternaires sont des sels d'un cation de formule

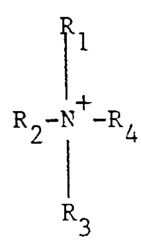

dans laquelle $R_1$, $R_2$, $R_3$ et $R_4$ représentent indépendamment un groupement $-CH_3$, $-CH_2-C_6H_5$ ou $-CH_2-CH_2OH$.

6. Procédé de préparation d'un dérivé d'uréthanne tel que défini dans l'une quelconque des revendications précédentes, caractérisé par le fait que l'on fait réagir un sel d'un acide aminé (D-,L-, ou DL)choisi parmi la sérine, la thréonine et la citrulline avec un composé de formule II

X-CO-OR' (II)

X représentant un halogène ou un groupement 1-imidazolyle, dans un solvant approprié, que l'on isole selon les méthodes connues le dérivé d'uréthanne correspondant de formule I formé et que, si désiré, on transforme ledit dérivé d'uréthanne en sel correspondant.

7. Composition cosmétique ou pharmaceutique, caractérisée par le fait qu'elle comprend comme ingrédient

actif au moins un dérivé d'uréthanne tel que défini dans l'une quelconque des revendications 1 à 5, dans un véhicule compatible avec l'administration sur la peau et/ou sur les cheveux.

**8.** Composition selon la revendication 7, caractérisé par le fait que la concentration dudit dérivé d'uréthanne est dans la gamme de 0,1 à 15% en poids par rapport au poids total de la composition.

**9.** Composition selon la revendication 8, caractérisé par le fait que ladite concentration du dérivé d'uréthanne est dans la gamme 0,5 à 5% en poids par rapport au poids total de la composition.

**10.** Utilisation d'un dérivé d'uréthanne tel que défini dans l'une quelconque des revendications 1 à 5 comme ingrédient actif dans la préparation d'une composition cosmétique ou pharmaceutique destinée au traitement ou au soin des peaux sèches.

**11.** Procédé de traitement cosmétique destiné notamment à améliorer l'aspect et l'élasticité de la peau des personnes à peau sèche, ou destiné à prévenir l'apparition des troubles esthétiques provoqués par ce phénomène de peau sèche, caractérisé par le fait que l'on applique sur la peau des parties du corps concernées, y compris éventuellement le cuir chevelu, une composition cosmétique telle que définie dans l'une quelconque des revendications 7 à 9.

## Patentansprüche

**1.** Urethanderivate der allgemeinen Formel:

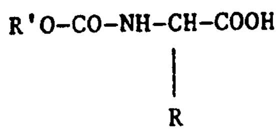

(I) ,

worin R eine Gruppe

$$-CH_2OH,\ -CHOH-CH_3 \text{ oder } -(CH_2)_3-NH-CO-NHY,$$

bedeutet,
Y -H oder COOR' bedeutet,
R' eine verzweigte oder lineare, gegebenenfalls ungesättigte Alkylgruppe mit 8 bis 24 Kohlenstoffatomen oder eine monocyclische Cycloalkylgruppe substituiert durch ein Alkyl, wobei die Gesamtzahl der Kohlenstoffatome gleich oder mehr als 10 ist, bedeutet,
sowie die Salze der Verbindungen der Formel I und die Mischungen der Verbindungen der Formel I und/oder der Salze.

**2.** Urethanderivate nach Anspruch 1, dadurch gekennzeichnet, daß die Salze Metallsalze oder Salze von organischen Kationen sind, die mit der Anwendung auf die Haut kompatibel sind.

**3.** Urethanderivate nach Anspruch 2, dadurch gekennzeichnet, daß die Salze aus den Natriumsalzen, Zinksalzen, Magnesium- und Aluminiumsalzen und den Kupfer (II) (-salzen) ausgewählt sind.

**4.** Urethanderivate nach Anspruch 2, dadurch gekennzeichnet, daß die Salze die Salze des quaternären Ammoniums sind.

**5.** Urethanderivate nach Anspruch 4, dadurch gekennzeichnet, daß die quaternären Ammoniumsalze Salze eines Kations der Formel

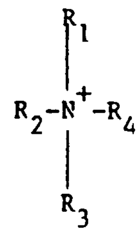

sind, in der $R_1$, $R_2$, $R_3$ und $R_4$ unabhängig voneinander eine Gruppe $-CH_3$, $-CH_2-C_6H_5$ oder $-CH_2-CH_2OH$ bedeuten.

6. Verfahren zur Herstellung eines Urethanderivates, wie in einem der vorhergehenden Ansprüche definiert, dadurch gekennzeichnet, daß man ein Salz einer Aminosäure (D-,L-, oder DL) ausgewählt aus Serin, Threonin und Citrulin mit einer Verbindung der Formel II

$$X\text{-}CO\text{-}OR' \qquad (II),$$

in der X ein Halogen oder eine 1-Imidazolylgruppe bedeutet, in einem geeigneten Lösungsmittel umsetzt, dann nach bekannten Verfahren die gebildeteten Urethanderivate entsprechend der Formel I isoliert, und, wenn gewünscht, die Urethanderivate in das entsprechende Salz überführt.

7. Kosmetische oder pharmazeutische Zusammensetzung, dadurch gekennzeichnet, daß sie als Wirkstoff mindestens ein Urethanderivat, wie in einem der vorhergehenden Ansprüche 1 bis 5 definiert, in einem mit der Verabreichung auf die Haut und/oder die Haare verträglichen Trägerstoff enthält.

8. Zusammensetzung nach Anspruch 7, dadurch gekennzeichnet, daß die Konzentration des Urethanderivates im Bereich von 0,1 bis 15 Gew.-%, bezogen auf das Gesamtgewicht der Zusammensetzung, liegt.

9. Zusammensetzung nach Anspruch 8, dadurch gekennzeichnet, daß die Konzentration des Urethanderivates im Bereich von 0,5 bis 5 Gew.-%, bezogen auf das Gesamtgewicht der Zusammensetzung, liegt.

10. Verwendung eines Urethanderivates wie in einem der vorhergehenden Ansprüche 1 bis 5 definiert, als Wirkstoff in der Herstellung einer kosmetischen oder pharmazeutischen Zusammensetzung für die Behandlung oder die Pflege der trockenen Haut.

11. Verfahren zur kosmetischen Behandlung, insbesondere zur Verbesserung des Aussehens und der Geschmeidigkeit, der Haut von Personen mit trockener Haut oder zur Verhinderung des Auftretens von ästhetischen Nachteilen ausgelöst durch das Phänomen der trockenen Haut, dadurch gekennzeichnet, daß man auf die Haut der betroffenen Körperteile, darin eingeschlossen ist gegebenenfalls die Kopfhaut, eine kosmetische Zusammensetzung wie in einem der vorhergehenden Ansprüche 7 bis 9 definiert, aufbringt.

## Claims

1. Urethane derivatives of general formula:

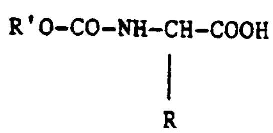

(I)

in which R represents a group

$-CH_2OH$, $-CHOH-CH_3$ or $-(CH_2)_3-NH-CO-NHY$,

Y representing -H or COOR',

R' represents a linear or branched alkyl group, optionally unsaturated, having from 8 to 24 carbon atoms, or a monocyclic cycloalkyl group substituted with an alkyl in which the total number of carbon atoms is not less than 10,

as well as salts of the compounds of formula I, and the mixtures of the compounds of formula I and/or their salts.

2. Urethane derivatives according to Claim 1, characterised in that the said salts are metal salts or salts of organic cations which are compatible with application to the skin.

3. Urethane derivatives according to Claim 2, characterised in that the said salts are chosen from the sodium, zinc, magnesium and aluminium salts and the cupric salts.

4. Urethane derivatives according to Claim 2, characterised in that the said salts are quaternary ammonium salts.

5. Urethane derivatives according to Claim 4, characterised in that the said quaternary ammonium salts are salts of a cation of formula

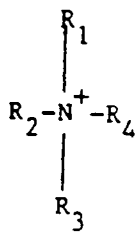

in which $R_1$, $R_2$, $R_3$ and $R_4$ independently represent a $-CH_3$, $-CH_2-C_6H_5$ or $-CH_2-CH_2OH$ group.

6. Process for preparing a urethane derivative as defined in any one of the preceding claims, characterised in that a salt of an amino acid (D-, L- or DL-), chosen from serine, threonine and citrulline, is reacted with a compound of formula II

X-CO-OR' (II)

X representing a halogen or a 1-imidazolyl group, in a suitable solvent, in that the corresponding urethane derivative of formula I formed is isolated according to known methods and that, if desired, the said urethane derivative is converted to a corresponding salt.

7. Cosmetic or pharmaceutical composition, characterised in that it comprises as active ingredient at least one urethane derivative as defined in any one of Claims 1 to 5, in a vehicle compatible with administration to the skin and/or to the hair.

8. Composition according to Claim 7, characterised in that the concentration of the said urethane derivative is within the range from 0.1 to 15 % by weight relative to the total weight of the composition.

9. Composition according to Claim 8, characterised in that the said concentration of the urethane derivative is within the range 0.5 to 5 % by weight relative to the total weight of the composition.

10. Use of a urethane derivative as defined in any one of Claims 1 to 5, as active ingredient in the preparation of a cosmetic or pharmaceutical composition intended for the treatment or care of dry skin.

11. Cosmetic treatment process intended, in particular, for improving the appearance and elasticity of the skin of individuals with dry skin, or intended for preventing the appearance of unsightly manifestations caused by this phenomenon of dry skin, characterised in that a cosmetic composition as defined in any one of Claims 7 to 9 is applied to the skin of the parts of the body which are affected, including, where appropriate, the scalp.

FIGURE UNIQUE